# EUROPEAN PATENT APPLICATION

(11) **EP 3 553 493 A1**
(43) Date of publication of application: **16.10.2019**
(21) Application number: 18166803.9
(22) Date of filing: 11.04.2018
(51) Int. Cl.: G01N 3/08

(54) **A METHOD AND A SYSTEM FOR DETERMINING A CROSSLINKING DEGREE OF A CROSSLINKED POLYMER PIPE**

(71) Applicant: Crosslink Finland Oy, 33540 Tampere (FI)
(72) Inventor: Heino, Aarne, 33540 Tampere (FI)
(74) Representative: Berggren Oy, Tampere

(57) **Abstract**

The disclosure relates to a method and a system for determining crosslinking degree of a crosslinked polymer pipe, which method and system enable determining a crosslinking degree of a crosslinked polymer pipe online, in a nondestructive manner. In the method, when the crosslinked polymer pipe is still in a melt state, a load is applied on the crosslinked polymer pipe. A resistive force by which the crosslinked polymer pipe resists deformation is measured, and the crosslinking degree of the crosslinked polymer pipe is determined based on the measured resistive force.

## Description

### Technical field

This specification relates to a method and a system for determining a crosslinking degree of a crosslinked polymer pipe. Some aspects of the disclosure relate to a method and a system for determining a crosslinking degree of a crosslinked polymer pipe by applying a load on the crosslinked polymer pipe and measuring a resistive force by which the crosslinked polymer pipe resists a deformation.

### Background

Crosslinking is a process of linking polymer chains to one another, thus causing a spatial network to be formed. In the synthetic polymer science field, crosslinking usually refers to the use of crosslinks to promote a difference in the polymers' physical properties. Crosslinked polyethylene (PEX) is a form of polyethylene with crosslinks. Crosslinks are intermolecular covalent bonds between the polyethylene polymer chains. Crosslinked bonds of the polymer structure enable change of a thermoplastic polymer to a thermoset polymer.

Main manufacturing processes for preparing crosslinked polymer pipes are peroxide method, silane method and electronic irradiation method. In crosslinking, a polymer that is to be crosslinked is mixed with an additive before extrusion. The crosslinking reaction may be triggered by heating.

In order to guarantee uniform and desired quality of the crosslinked polymer pipes manufactured, the crosslinking degree of the pipes has to be monitored. The required degree of crosslinking according to ASTM Standard F876 is between 65 and 89 %. A higher degree of crosslinking could result in brittleness and stress cracking of the material, while a lower degree of crosslinking could result in a product with poorer physical properties. Crosslinking degree may be measured by swelling experiments. Crosslinking degree of a ready pipe may be determined by rubbing out a sample of the pipe. The sample is placed into a suitable solvent at a specific temperature, and either the change in mass or the change in volume is measured. The more crosslinking, the less swelling is attainable. Two ASTM standards are commonly used to describe the degree of crosslinking in thermoplastics. In ASTM D2765, the sample is weighed, then placed in a solvent for 24 hours, weighed again while swollen, then dried and weighed a final time. The degree of swelling and the soluble portion can be calculated. In another ASTM standard, F2214, the sample is placed in an instrument that measures the height change in the sample, allowing the user to measure the volume change. The crosslink density can then be calculated.

Another method typically used in relation to crosslinked pipes is to cut a sample of a pipe and measure time that is needed for a certain radiation to proceed through the sample. The time is proportional to the crosslinking degree of the sample. The method is specified in a standard DIN 16892.

The above disclosed methods for determining crosslinking degree of a polymer pipe are destructive, meaning that the structure of the pipe is broken down when implementing the method.

### Summary

The above disclosed deficiencies may be addressed by providing a method and a system which enable determining a crosslinking degree of a crosslinked polymer pipe online, and furthermore, without causing the crosslinked polymer pipe to be destructed. The method and the system allow real-time measurement of the crosslinking degree during the manufacturing process. An advantage is, that in a case any defect in the manufacturing process affecting the crosslinking degree of the manufactured pipe is observed, it is possible to react to it in a rapid manner. This prevents unnecessary manufacturing of crosslinked products of poor or unwanted quality. A further advantage is that, since the crosslinking degree is determined without causing the pipe to be destructed, the pipe portion used for the determination of the crosslinking degree may later on be used normally, thus diminishing waste of the material. Further, the disclosure provides a solution that is of simple design and technique, as well as cost-efficient.

According to an aspect of the disclosure, subsequently after the crosslinking oven, when the crosslinked polymer pipe is still in a melt state, the crosslinked polymer pipe is arranged to proceed between a lower support and an upper support, and a load is applied on the crosslinked polymer pipe. The load applied may be directed radially on a portion of the crosslinked polymer pipe. Magnitude of the load applied on the crosslinked polymer pipe is preferably such that no deformation at all, or as small deformation as possible of the crosslinked polymer pipe is obtained. The load is applied only to such an extent that the diameter of the crosslinked polymer pipe remains substantially the same. A resistive force by which the crosslinked polymer pipe resists deformation is measured by a force sensor mounted to the upper support. The crosslinking degree of the crosslinked polymer pipe is determined based on the measured resistive force.

Therefore, there is provided a method for determining a crosslinking degree of a crosslinked polymer pipe, wherein the method comprises
- applying a load on the crosslinked polymer pipe, the crosslinked polymer pipe being in a melt state,
- measuring a resistive force by which the crosslinked polymer pipe resists deformation, and
- determining the crosslinking degree of the crosslinked polymer pipe based on the measured resistive force.

Further, there is provided a system for determining a crosslinking degree of a crosslinked polymer pipe, wherein the system comprises
- a lower support,
- an upper support, and
- a force sensor mounted to the upper support.

Objects according to this disclosure are further described in the appended claims.

### Brief description of the drawings

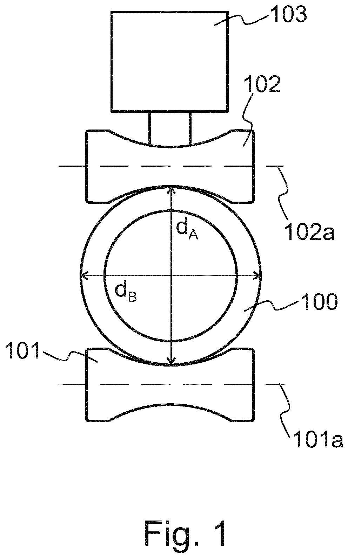

- Figure 1: illustrates, by way of an example, a cross-sectional view of a system according to an embodiment when viewed from the propagation direction of the crosslinked polymer pipe.

The figure is schematic, and is not in any particular scale.

### Detailed description

In this specification, a pipe stands for a hollow elongated tube or cylinder for conducting for example a liquid, a gas, a finely divided solid or a combination thereof.

Crosslinked polymer pipe refers to a polymer pipe which may have been crosslinked in its entirety or which may comprise a polymer layer that has been crosslinked.

Machine direction refers to a direction in which the pipe proceeds during manufacturing process. Machine direction is from extruder to the means for cooling the produced pipe.

A crosslink is a bond that links one polymer chain to another. Crosslinks are formed by chemical reactions that may be initiated for example by heat, pressure, change in pH, or radiation. The degree of crosslinking relates to the number of groups that interconnect polymer chains. The degree of crosslinking is generally expressed in mole percent.

By crosslinking a spatial network is formed. In synthetic polymer science field, crosslinking usually refers to a the use of crosslinks to promote a difference in the polymers' physical properties. Crosslinked polyethylene (PEX) is a form of polyethylene with crosslinks. PEX contains crosslinked bonds in the polymer structure, changing the thermoplastic polymer to a thermoset polymer. There are three main manufacturing processes for preparing crosslinked polymer pipes: peroxide method, silane method and electronic irradiation method. According to DIN 16892 standard setting up quality requirements for pipes made of PEX at least following degrees of crosslinking must be achieved: in peroxide crosslinking 75 %, with silane crosslinking 65 % and with electronic irradiation crosslinking 60 %.

Typically, the crosslinked polymer pipe is manufactured in a system that comprises an extruder, a crosslinking oven and means for cooling the polymer pipe, for example a cooling tub. The extruder comprises an extruder head. The extruder head may be located outside the crosslinking oven, and may be arranged to open into said oven. Typically, the crosslinking oven comprises at least one heating unit.

In manufacturing crosslinked polymer pipes, the starting materials are fed in the extruder. The starting materials comprise a polymer and a crosslinking additive. The starting materials may comprise for example polyethylene and organic peroxide. The starting materials are heated and mixed in the extruder, whereafter the polymer pipe is continuously formed in the extruder head. Polymer material is extruded in form of a pipe from the extruder head. The formed polymer pipe is then forwarded to the crosslinking oven. In the crosslinking oven the polymer pipe is arranged to proceed through at least one heating unit. Heating of the polymer pipe by the at least one heating unit may be implemented for example by a heating tool (conduction), a heat transfer from a liquid, hot air or infrared radiation. From the crosslinking oven the polymer pipe is arranged to proceed to cooling. Cooling of the polymer pipe terminates the crosslinking reaction taking place in the polymer material. Cooling may be carried out for example by cold water or water spray.

As presented above, in order to guarantee uniform and desired quality of the crosslinked polymer pipe manufactured, the crosslinking degree of the pipe has to be monitored. It is preferred to monitor the crosslinking degree online in order to obtain real-time data on the process. Within context of this specification, online means that the monitoring is performed while the manufacturing process is on the way. Thus, there is no need to interrupt the manufacturing process in order to enable controlling the crosslinking degree of the formed pipe. The manufacturing process is a continuous process. This has the effect that in a case any defect in the manufacturing process affecting the crosslinking degree of the manufactured pipe is observed, it is possible to react to it in a rapid manner. In case a defect in the manufacturing process is observed, the process may be stopped until necessary measure(s) for improving the process are performed. Alternatively, it may be possible to perform the necessary measures without stopping the manufacturing process. Reacting rapidly to the defects in the manufacturing process prevents unnecessary manufacturing of the crosslinked products of poor or unwanted quality. The defects in the manufacturing process may relate for example to the feed of the crosslinking additive or to the crosslinking process itself.

When determining the crosslinking degree of the polymer pipe online, it is desirable to use a method which does not cause the structure of the formed pipe to break down or to be deformed. This has the effect, that the pipe portion used for the determination of the crosslinking degree may be used normally, thus diminishing waste of material.

Crosslinking degree of the polymer pipe is related to the ring stiffness of the polymer pipe. Ring stiffness of the pipe refers to its force-deformation behavior under a radially acting external mechanical load. Ring stiffness corresponds to a gradient in the force-deformation diagram. Higher crosslinking degree corresponds to higher ring stiffness.

Deformation refers to any changes in the shape or size of an object. Deformation may be caused by an applied force or by a change in temperature. Deformation may also be described as strain. As deformation occurs, internal intermolecular forces arise that oppose the applied force. If the applied force is not too great, these internal intermolecular forces may be sufficient to completely resist the applied force and allow the object to assume a new equilibrium state and to return to its original state when the load is removed. A larger applied force may lead to a permanent deformation of the object or even to its structural failure.

A crosslinked polymer pipe 100 is produced by a crosslinking process. In the crosslinking process polymer material is mixed and heated in an extruder. The polymer material is extruded from an extruder head in form of the polymer pipe. After the extruder, the polymer pipe is heated in a crosslinking oven for the crosslinking in the polymer material of the pipe to take place.

A method for determining a crosslinking degree of a crosslinked polymer pipe 100 is disclosed. In the method, subsequently after the crosslinking oven, when the crosslinked polymer pipe 100 is still in a melt state, a load is applied on the crosslinked polymer pipe 100. The load applied may be directed radially on a portion of the crosslinked polymer pipe 100. The melt state of the crosslinked polymer pipe 100 refers to a state wherein the temperature of the crosslinked polymer pipe 100 is above the melting point of the polymer material. The load applied may be for example a compressive force. Magnitude of the load applied on the crosslinked polymer pipe 100 is preferably such that no deformation at all, or as small deformation as possible of the crosslinked polymer pipe 100 is obtained. This may be controlled by monitoring a diameter d_{A}, d_{B} of the crosslinked polymer pipe 100. The load is applied only to such an extent that the diameter d_{A}, d_{B} of the crosslinked polymer pipe 100 remains substantially the same, that is to say, does not change or changes less than 1 %. The load is applied only to such an extent that a ratio of a vertical diameter d_{A} and a horizontal diameter d_{B} of the crosslinked polymer pipe is substantially 1, or at least does not deviate from 1 more than 0.01 %.

A resistive force by which the crosslinked polymer pipe 100 resists the deformation is measured. The resistive force by which the crosslinked polymer pipe 100 resists the deformation may correspond to the compressive strength of the crosslinked polymer pipe. The resistive force measured is compared to a library comprising resistive force values for reference polymer pipes having known crosslinking degrees. The crosslinking degree of the crosslinked polymer pipe 100 is determined based on the comparison of the resistive force values.

According to an embodiment, the crosslinked polymer pipe 100 in the melt state is arranged to proceed between a lower support and an upper support. While the crosslinked polymer pipe 100 proceeds between the lower support and the upper support, a load is applied on a portion of the crosslinked polymer pipe. The length of the portion of the pipe on which the load is applied may be equal to or less than a width of the upper support in contact with the longitudinal direction of the pipe. The resistive force by which the crosslinked polymer pipe 100 resists deformation is effected to the upper support. The resistive force effected to the upper support is measured by a force sensor 103 mounted to the upper support.

According to an embodiment illustrated in Figure 1, the crosslinked polymer pipe 100 in a melt state is arranged to proceed between a lower support roll 101 and an upper support roll 102. The lower support roll 101 and the upper support roll 102 are arranged to roll around their longitudinal axles 101 a and 102a, respectively, while the crosslinked polymer pipe 100 proceeds between the support rolls. The crosslinked polymer pipe 100 is arranged to proceed above the lower support roll 101 and below the upper support roll 102. The lower support roll 101 and the upper support roll 102 are arranged such that a load is applied by them on the crosslinked polymer pipe 100. The resistive force by which the crosslinked polymer pipe 100 resists deformation is effected to the upper support roll 102. The resistive force effected to the upper support roll 102 is measured by a force sensor 103 mounted to the upper support roll 102.

A system for determining a crosslinking degree of a crosslinked polymer pipe 100 comprises a lower support, an upper support and a force sensor 103 mounted to the upper support. The lower support and the upper support are adapted to apply a load on the crosslinked polymer pipe 100. The force sensor 103 is adapted to measure forces effected to the upper support. The force sensor 103 may be a sensor for measuring for example shear force, tension and/or compression. The force sensor 103 may be for example a load pin.

The load pin typically senses a force applied across it, via strain gauges installed within a small bore through a center of the pin. Under applied load or pressure an electrical output signal proportional to the strain is generated when excited by a regulated voltage or current source. This signal is usually only a few millivolts and may require amplification before it can be read.

Surfaces of the upper support and the lower support arranged to be in touch with the crosslinked polymer pipe 100 may comprise a low-friction material, such as polytetrafluoroethylene. The lower support may be the lower support roll 101 and the upper support may be the upper support roll 102.

The system comprising the lower and upper supports as well as the force sensor 103 mounted to the upper support is of a simple design and technique, and thus easily and cost-efficiently implementable to existing manufacturing systems.

The system may comprise means for storing reference resistive force values obtained for reference polymer pipes having known crosslinking degrees. Within context of this specification, this is called a library. The library is established by measuring resistive forces by which crosslinked pipes having known crosslinking degrees resist deformation, and storing the measured resistive force values into a database.

The system may comprise means, for example software means, for comparing the measured resistive force to reference resistive force values obtained for reference polymer pipes having known crosslinking degrees, namely, the library values, and for determining the crosslinking degree of the crosslinked polymer pipe 100 based on comparison of the resistive force values.

The system for determining a crosslinking degree of a crosslinked polymer pipe 100 may comprise display means for displaying values related to the crosslinking degree of the crosslinked polymer pipe 100. The value displayed may be for example the force measured by the force sensor or the crosslinking degree of the crosslinked polymer pipe 100 determined based on comparison of the measured resistive force and the library value.

The system may also comprise means for generating an alarm in order to alert personnel operating the system in case the measured resistive force and/or the crosslinking degree of the produced crosslinked polymer pipe 100 does not fit into a predetermined range. The alarm may be for example an audible signal and/or a light signal. This allows the personnel operating the manufacturing process to be more easily aware of any occasion in the manufacturing process that might require their attention.

A method for determining a crosslinking degree of a crosslinked polymer pipe is provided. The method comprises, applying a load on the crosslinked polymer pipe, the crosslinked polymer pipe being in a melt state, measuring a resistive force by which the crosslinked polymer pipe resists deformation, and determining the crosslinking degree of the crosslinked polymer pipe based on the measured resistive force. The crosslinked polymer pipe may be arranged to proceed between a lower support and an upper support, and the resistive force effected to the upper support may be measured. The lower support may be a lower support roll and the upper support may be an upper support roll. The method may further comprise comparing the measured resistive force to reference resistive force values obtained for reference polymer pipes having known crosslinking degrees. The method may be implemented online while manufacturing the crosslinked polymer pipe. The method may further comprise generating an alarm based on the measured resistive force and/or the crosslinking degree.

A system for determining a crosslinking degree of a crosslinked polymer pipe is provided. The system comprises a lower support, an upper support, and a force sensor mounted to the upper support. The force sensor may be adapted to measure resistive forces effected to the upper support. The lower support may be a lower support roll and the upper support may be an upper support roll. The force sensor may be a sensor for measuring shear force. The force sensor may be a load pin. The system may further comprise means for comparing the measured resistive force to reference resistive force values obtained for reference polymer pipes having known crosslinking degrees. The system may further comprise means for displaying values related to the crosslinking degree of the crosslinked polymer pipe. The system may further comprise means for generating an alarm.

Many obvious variations of the embodiments set out herein will suggest themselves to those skilled in the art in light of the present disclosure. Such obvious variations are within the full intended scope of the appended claims.

## Claims

1. A method for determining a crosslinking degree of a crosslinked polymer pipe (100), wherein the method comprises
- applying a load on the crosslinked polymer pipe (100), the crosslinked polymer pipe (100) being in a melt state,
- measuring a resistive force by which the crosslinked polymer pipe (100) resists deformation, and
- determining the crosslinking degree of the crosslinked polymer pipe (100) based on the measured resistive force.

2. A method according to claim 1, wherein the method comprises
- arranging the crosslinked polymer pipe (100) to proceed between a lower support and an upper support, and
- measuring the resistive force effected to the upper support.

3. A method according to claim 1 or 2, wherein the lower support is a lower support roll (101) and the upper support is an upper support roll (102).

4. A method according to any of the previous claims, wherein the method further comprises comparing the measured resistive force to reference resistive force values obtained for reference polymer pipes having known crosslinking degrees.

5. A method according to any of the previous claims, wherein the method is implemented online while manufacturing the crosslinked polymer pipe (100).

6. A method according to any of the previous claims, wherein the method further comprises generating an alarm based on the measured resistive force and/or the crosslinking degree.

7. A system for determining a crosslinking degree of a crosslinked polymer pipe (100), wherein the system comprises
- a lower support,
- an upper support, and
- a force sensor (103) mounted to the upper support.

8. A system according to claim 7, wherein the force sensor (103) is adapted to measure resistive forces effected to the upper support.

9. A system according to claim 7 or 8, wherein the lower support is a lower support roll (101) and the upper support is an upper support roll (102).

10. A system according to any of claims 7-9, wherein the force sensor (103) is a sensor for measuring shear force.

11. A system according to any of claims 7-10, wherein the force sensor (103) is a load pin.

12. A system according to any of claims 7-11, wherein the system further comprises means for comparing the measured resistive force to reference resistive force values obtained for reference polymer pipes having known crosslinking degrees.

13. A system according to any of claims 7-12, wherein the system further comprises means for displaying values related to the crosslinking degree of the crosslinked polymer pipe (100).

14. A system according to any of claims 7-13, wherein the system further comprises means for generating an alarm.
